# EUROPÄISCHE PATENTSCHRIFT

(11) **EP 1 981 467 B1**
(45) Veröffentlichungstag und Bekanntmachung des Hinweises auf die Patenterteilung: **08.07.2009**
(21) Anmeldenummer: 07701311.8
(22) Anmeldetag: 09.02.2007
(51) Int. Cl.: A61J 9/00, B65D 75/66, B65D 75/44

(54) **GEFRIERBEUTEL**
FREEZER BAG
SAC CONGÉLATION

(30) Priorität: 10.02.2006 AT 2112006
(43) Veröffentlichungstag der Anmeldung: 22.10.2008
(73) Patentinhaber: Mam Babyartikel Gesellschaft m.b.H., 1160 Wien (AT)
(72) Erfinder: RÖHRIG, Peter, 1160 Wien (AT)
(74) Vertreter: Sonn & Partner Patentanwälte
(86) Internationale Anmeldenummer: PCT/AT2007/000067
(87) Internationale Veröffentlichungsnummer: WO 2007/090222

(56) Entgegenhaltungen:
- CA-A1- 2 300 297
- DK-B- 154 060
- US-A- 4 223 043
- US-A- 4 783 042
- US-A1- 2002 156 419
- ANONYM: "Muttermilch in Eiswürfeltüten" INTERNET ARTIKEL, [Online] 14. Oktober 2000 (2000-10-14), XP002435188 Gefunden im Internet: URL:http://www.ciao.de/Milchpumpen_Tipps_T ricks__Test_1227523> [gefunden am 2007-05-25]

## Beschreibung

Die Erfindung betrifft einen Muttermilch-Gefrierbeutel zur Aufnahme und Aufbewahrung von Muttermilch mit einer Einfüllöffnung, wobei die Einfüllöffnung einen sich über den oberen Rand des Muttermilch-Gefrierbeutels erstreckenden nach außen umklappbaren Kragen zur Befestigung des Muttermilch-Gefrierbeutels mit einer Brustpumpe aufweist.

Muttermilch-Gefrierbeutel sind Behältnisse für Muttermilch aus flexiblem, dünnschichtigem Material, die für die Abfüllung von frischer Muttermilch und ihrer Kühlung oder Tiefkühlung konzipiert sind.

Ein derartiger Muttermilch-Gefrierbeutel mit einer einzigen Aufnahmeeinheit zur Aufnahme von Muttermilch ist aus der US 2002/0156419 A1 bekannt. Dieser Gefrierbeutel kann mit einer Brustpumpe verbunden werden, wobei er hierfür einen speziell ausgebildeten oberen Rand aufweist, so dass die mit Hilfe der Brustpumpe abgepumpte Muttermilch direkt in die Aufnahmeeinheit eingefüllt werden kann.

Einem im Internet veröffentlichten Artikel
(URL:http://www.ciao.de./Milchpumpe_Tipps_Tricks_Test_1227523, gefunden am 2007-05-25), ist lediglich der allgemeine Hinweis zu entnehmen, herkömmliche Einwegtüten für Eiswürfel zu verwenden, um diese mit Muttermilch zu befüllen.

Weitere Muttermilch-Gefrierbeutel mit einer einzigen Aufnahmeeinheit sind aus der CA 2 300 297 A1, der JP 08 198 279 A und der JP 2004-010155 A bekannt. Diese Gefrierbeutel werden nach Abfüllung der Milch eingefroren und zur Fütterung des Babys entweder noch gefroren oder aufgetaut in eine Milchflasche eingebracht, wobei sie einen speziellen Öffnungs- und Schließmechanismus aufweisen, der ein dichtes Verschließen und ein leichtes Öffnen des Beutels ermöglicht. Die CA 2 300 297 A1, weist hierzu einen Verschluss in der Form eines einfachen Bandes bzw. eines mit Kunststoff überzogenen dünnen Drahts auf, der vom Beutel gelöst werden kann. Somit kann der Beutel mit einer Brüstpumpe auf aufwändige Weise verbunden werden, indem die Verschlussbänder um eine Auslassöffnung umwickelt werden und die beiden Enden der Befestigungsbänder miteinander verknüpft werden.

Der Muttermilch-Gefrierbeutel gemäß der US 6 899 239 B1 zeigt darüber hinaus einen Schnappverschluss, um die Aufnahmeeinheit bzw. die Tasche zur Aufnahme der abgepumpten Muttermilch bei nicht vollständiger Verfütterung der darin enthaltenen Milch zu verschließen und so den verbleibenden Rest der Muttermilch aufzubewahren.

Die DK 154 060 B zeigt einen andersartigen Einwegbeutel zum Frieren von Eis aus flüssigen Nahrungsmitteln für den Heim- bzw. Hausgebrauch. Der Beutel besteht aus Kunststoffmaterial mit horizontalen, länglichen Räumen, die über Durchgänge kommunizierend miteinander verbunden sind. Der Beutel wird mit verdünntem, aufgespritzten Fruchtsaft für den menschlichen Verzehr befüllt und eingefroren, wonach die mit gefrorenem Fruchtsaft gefüllten Räume voneinander getrennt und als Eisschlecker durch Abziehen oder Abreißen eines Teils des Formstoffmaterials verzehrt werden können. Der nicht abgezogene Teil des Formstoffmaterials dient als Hülle, zum Halten des Eisschleckers.

Aus der JP 2001-299905 A ist eine Brustpumpe mit einer Milchflasche und ein eigens für diese Milchflasche konzipierter Milchaufbewahrungsbehälter bekannt. Auch dieser spezielle Aufbewahrungsbehälter weist lediglich einen Aufnahmebereich für die Muttermilch auf, wobei die Einfüllöffnung des Behälters in Form eines Konus zur Verbindung mit der Brustpumpe geformt ist.

Nachteilig ist bei all diesen bekannten Muttermilch-Gefrierbeuteln, dass sie ausschließlich zur Aufnahme von größeren Mengen an Milch vorgesehen sind. Wenn nach der tiefgekühlten Aufbewahrung nur eine kleinere Menge Milch benötigt wird, muss der gesamte Beutel aufgetaut bzw. gewärmt werden. Die nicht benötigte Milch verbleibt unverwendet im Beutel und muss entsorgt werden bzw. binnen kürzester Zeit aufgebraucht werden, da sie ansonsten aus hygienischen und gesundheitlichen Gründen für das Baby nicht mehr verwendbar ist. Derartige Muttermilch-Gefrierbeutel sind demzufolge, insbesondere für Mütter, die nur über eine relativ geringfügige Menge an Muttermilch verfügen, nachteilig, da ein wesentlicher Teil der abgepumpten Muttermilch nach dem Auftauen ungenutzt entsorgt werden muss.

Ein andersartiger Wegwerfbeutel zur Aufnahme von Milchpulver und Wasser ist aus der US 2 885 104 A bekannt. Dieser Wegwerfbeutel ist in zwei durch eine Schweißnaht getrennte Abschnitte unterteilt, die einerseits zur Aufnahme von Wasser und andererseits von Milchpulver vorgesehen sind. Die zwei in Betriebsstellung übereinander angeordneten Abschnitte können mittels eines Streifens, der bei der Herstellung des Einsatzes in die Trennnaht miteingeformt wird, zu einem einheitlichen Abschnitt umgestaltet werden. Hiefür wird am unteren Ende des Streifens, das durch den zum Teil offenen Boden der Babyflasche durchgeführt ist, angezogen. Dadurch wird die Trennnaht der beiden Abschnitte gelöst bzw. aufgetrennt, so dass das im oberen Abschnitt befindliche Wasser in den unteren Abschnitt mit dem Milchpulver gelangt.

Weiters sind grundsätzlich bereits andersartige Gefrierbeutel bekannt, die in mehrere Aufnahmeeinheiten unterteilt sind. Derartige Gefrierbeutel, wie sie beispielsweise in der US 4 783 042 A, der DK 104 681 A und der US 4 223 043 A geoffenbart sind, sind jedoch nicht zur Verbindung mit einer Brustpumpe geeignet und zudem lediglich vorgesehen um tiefgefrorene Eislutscher bzw. gefrorenes Konfekt herzustellen, welche einzeln im gefrorenen Zustand verzehrt bzw. geschleckt werden können.

Demgegenüber ist es nun Aufgabe der Erfindung einen Muttermilch-Gefrierbeutel der eingangs genannten Art zur Verfügung zu stellen, der es ermöglicht, je nach Bedarf unterschiedliche Mengen an Muttermilch auf einfache Weise zur Verfügung zu stellen, ohne dass hierbei die gesamte im Muttermilch-Gefrierbeutel aufgenommene Muttermilch aufgetaut werden muss oder die abgepumpte Muttermilch umgefüllt werden muss.

Der Muttermilch-Gefrierbeutel ist erfindungsgemäß dadurch gekennzeichnet, dass wenigstens zwei Aufnahmeeinheiten, deren Aufnahmevolumen jeweils etwa 10 ml bis 50 ml beträgt, vorgesehen sind,
wobei die Aufnahmeeinheiten mit jeweils zumindest einer benachbarten Aufnahmeeinheit über zumindest einen Verbindungskanal kommunizierend verbunden sind, so dass die in den Aufnahmeeinheiten aufgenommene Muttermilch im eingefrorenen Zustand in Portionen entsprechend der Aufnahmevolumina der Aufnahmeeinheiten aufteilbar ist und im aufgetauten Zustand portionsweise verfütterbar ist.

Aufgrund der Unterteilung in wenigstens zwei Aufnahmeeinheiten, die lediglich über zumindest einen Verbindungskanal miteinander verbunden sind, kann die aufbewahrte Muttermilch in kleinere Portionen entsprechend der Aufnahmevolumina der Aufnahmeeinheiten aufgeteilt werden. Hierbei können die lediglich über die im Verbindungskanal in Verbindung stehenden eingefrorenen Muttermilchportionen auf einfache Weise voneinander getrennt werden, z.B. indem eine in einer Aufnahmeeinheit eingefrorene Portion Muttermilch aus der Aufnahmeeinheit hinaus gedrückt wird. Somit kann die gesamte im Muttermilch-Gefrierbeutel aufgenommene Milch auch tatsächlich portionsweise an ein Baby verfüttert werden und es ist nicht erforderlich, aufgetaute Muttermilch unverbraucht zu entsorgen.

Wenn die Aufnahmeeinheiten entlang einer den Verbindungskanal durchtrennenden Ebene voneinander trennbar sind, können die Aufnahmeeinheiten je nach Bedarf der Menge an Milch, d.h. ein oder mehrere Aufnahmeeinheiten, von den übrigen Aufnahmeeinheiten im tiefgefrorenen Zustand, z.B. mittels Reißen oder Schneiden, getrennt werden, so dass auf einfache Weise lediglich so viel Milch aufgetaut werden kann, wie auch tatsächlich benötigt wird.

Hinsichtlich einer einfachen Herstellung von voneinander getrennten Aufnahmeeinheiten, die miteinander über einen Verbindungskanal verbunden sind, ist es günstig, wenn der Muttermilch-Gefrierbeutel aus zwei Kunststoffflächen besteht, die unter Aussparung der Einfüllöffnung, der Aufnahmeeinheiten und der Verbindungskanäle flächig miteinander verschweißt sind. Die Kunststoffflächen sind vorzugsweise flexible, transparente oder färbige, vorteilhafterweise aber im Wesentlichen durchscheinende, Dünnschichtfolien, die zur hygienischen, insbesondere bakterienfreien, Aufbewahrung und zum Einfrieren von Muttermilch geeignet sind. Dabei ist es denkbar, eine Vielzahl von miteinander über ihren in Befüllstellung oberen und unteren Rand verschweißten Muttermilch-Gefrierbeuteln in Form eines Strangs oder Bands für ihren Vertrieb bereitzustellen, wobei der einzelne Muttermilch-Gefrierbeutel von dem nächsten Muttermilch-Gefrierbeutel mit Hilfe einer Perforation leicht trennbar gestaltet ist.

Um die Aufnahmeeinheiten des Muttermilch-Gefrierbeutels auf einfache Weise voneinander trennen zu können, z.B. mittels Schneiden, ist es vorteilhaft, wenn die Aufnahmeeinheiten über unter Aussparung der Verbindungskanäle flächig verschweißte Verbindungsabschnitte miteinander verbunden sind.

Um eine Aufnahmeeinheit von ihrer benachbarten Aufnahmeeinheit auf handliche Weise trennen zu können, ist es vorteilhaft, wenn die Verbindungsabschnitte Perforationen aufweisen. Die Abtrennung kann weiters erleichtert werden, wenn die Verbindungsabschnitte in zumindest einem Randabschnitt jeweils eine Trennkerbe aufweisen.

Hinsichtlich eines zuverlässigen Übertritts zwischen den Aufnahmeeinheiten beim Befüllen des Muttermilch-Gefrierbeutels mit Muttermilch sowie auch in Bezug auf ein einfach gestaltetes Trennen der einzelnen benachbarten Aufnahmeeinheiten ist es zweckmäßig, dass die Aufnahmeeinheiten in der Befüllstellung des Muttermilch-Gefrierbeutels übereinander angeordnet sind.

Um das Aufnahmevolumen des Muttermilch-Gefrierbeutels möglichst gut auszunutzen, ist es günstig, wenn die Aufnahmeeinheiten im Wesentlichen zylindrisch ausgebildet sind.

In diesem Zusammenhang ist es für eine möglichst effiziente Ausnutzung des zur Verfügung stehenden Aufnahmevolumens vorteilhaft, wenn die Grund- und Deckflächen der im Wesentlichen zylindrischen Aufnahmeeinheiten parallel zueinander angeordnet sind.

Um beim Abfüllen der Milch einen raschen und effizienten Übertritt zwischen den Aufnahmeeinheiten zu ermöglichen, ist es günstig, wenn die Verbindungskanäle zwischen den Aufnahmeeinheiten in der Längsachse des im Wesentlichen zylindrischen Muttermilch-Gefrierbeutels angeordnet sind.

Damit die Milch ohne Erschwernisse in den Muttermilch-Gefrierbeutel gefüllt werden kann und sich gleichmäßig in den Aufnahmeeinheiten verteilen kann, ist es zweckmäßig, dass die Einfüllöffnung vom oberen Rand des Gefrierbeutels in die vom oberen Rand des Gefrierbeutels gesehene erste bzw. oberste Aufnahmeeinheit mündet.

Durch das Vorsehen des entsprechend ausgebildeten Kragens am oberen Rand des Muttermilch-Gefrierbeutels kann der Gefrierbeutel auf einfache Weise mit Hilfe einer Kappe in der Art einer Überwurfmutter an einem mantelförmigen Gehäuse einer Brustpumpe befestigt werden. Wenn nun die Einfüllöffnung trichterförmig ausgebildet ist, fließt die abgepumpte Muttermilch zuverlässig in eine erste Aufnahmeeinheit. Je nach Art der zu verwendenden Brustpumpe, kann die Trichterform unterschiedlich gestaltet sein. Sie kann in ihrem breitesten Durchmesser nahe des in Befüllstellung des Muttermilch-Gefrierbeutels oberen Randes unterschiedlich groß dimensioniert sein. Ein trichterförmiger Verbindungsstutzen einer Brustpumpe für den Milchauslass aus der Pumpe kann somit direkt mit der Einfüllöffnung des Muttermilch-Gefrierbeutels, in Kontakt gebracht werden.

Hinsichtlich einer effizienten Ausnutzung des Aufnahmevolumens des Muttermilch-Gefrierbeutels ist es alternativ ebenso möglich, dass seitlich nebeneinander angeordnete Aufnahmeeinheiten vorgesehen sind, deren Längsachsen in der Befüllstellung im Wesentlichen lotrecht angeordnet sind. Hierbei ist es, um einen Übertritt von Muttermilch zwischen den Aufnahmeeinheiten auf kürzestem Weg zu ermöglichen, von Vorteil, wenn die Verbindungskanäle zwischen den Aufnahmeeinheiten senkrecht zur Längsachse der Aufnahmeeinheiten angeordnet sind.

Um eine gleichmäßige Befüllung der nebeneinander angeordneten Aufnahmeeinheiten mit Muttermilch zu ermöglichen, ist es günstig, wenn die Einfüllöffnung mit allen seitlich nebeneinander angeordneten Aufnahmeeinheiten in Verbindung steht.

Es hat sich gezeigt, dass bei einem Aufnahmevolumen der Aufnahmeeinheiten von etwa 20 ml bis etwa 40 ml, insbesondere etwa 30 ml, eine zweckmäßige Portionierung von Milchmahlzeiten für ein Baby gegeben ist.

Für die Verwendung der Milch aus dem Muttermilch-Gefrierbeutel ist es günstig, zumindest eine Aufschriftfläche an einer der Außenseiten des Gefrierbeutels vorzusehen, wodurch der Muttermilch-Gefrierbeutel mit dem Datum und/oder der Uhrzeit der Abfüllung der Milch gekennzeichnet werden kann.

In Bezug auf eine einfache Herstellung und die Haltbarkeit ist es von Vorteil, wenn der Muttermilch-Gefrierbeutel aus thermoplastischem Kunststoffmaterial besteht.

Die Erfindung wird nachstehend anhand von in den Zeichnungen dargestellten Ausführungsbeispielen näher erläutert. Dabei zeigen:
Fig. 1 und Fig. 2 jeweils eine Ansicht eines Muttermilch-Gefrierbeutels;
Fig. 3 eine auseinandergezogene Darstellung einer Brustpumpen-Anordnung mit einem Muttermilch-Gefrierbeutel gemäß Fig. 1 und Fig. 2;
Fig. 4 eine zusammengesetzte Darstellung der Brustpumpen-Anordnung gemäß Fig. 3;
Fig. 5 eine zusammengesetzte Darstellung der Brustpumpe mit einem alternativen Ausführungsbeispiel des Muttermilch-Gefrierbeutels;
Fig. 6 eine Ansicht eines Muttermilch-Gefrierbeutels mit seitlich nebeneinander angeordneten Aufnahmeeinheiten;
Fig. 7 eine Seitenansicht des Muttermilch-Gefrierbeutels gemäß Fig. 6; und
Fig. 8 einen Schnitt gemäß der Linie VIII-VIII in Fig. 6.

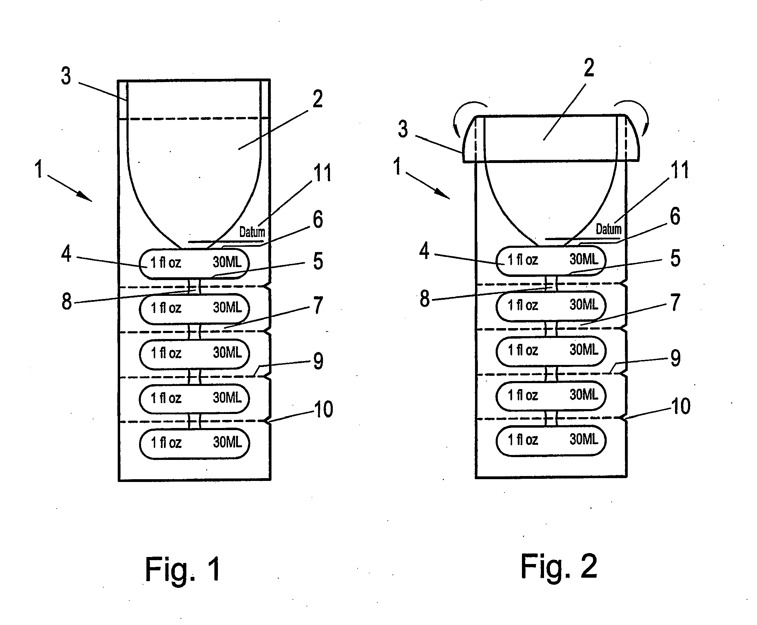

In Fig. 1 ist ein erfindungsgemäßer Muttermilch-Gefrierbeutel 1 zur Aufnahme und Aufbewahrung von Muttermilch gezeigt, der mehrere Aufnahmeeinheiten 4 aufweist. Der Muttermilch-Gefrierbeutel 1 hat eine im Wesentlichen zylindrische Form und besteht aus zwei zum Teil aneinander geschweißten Kunststofffolien, z.B. aus thermoplastischem Material.

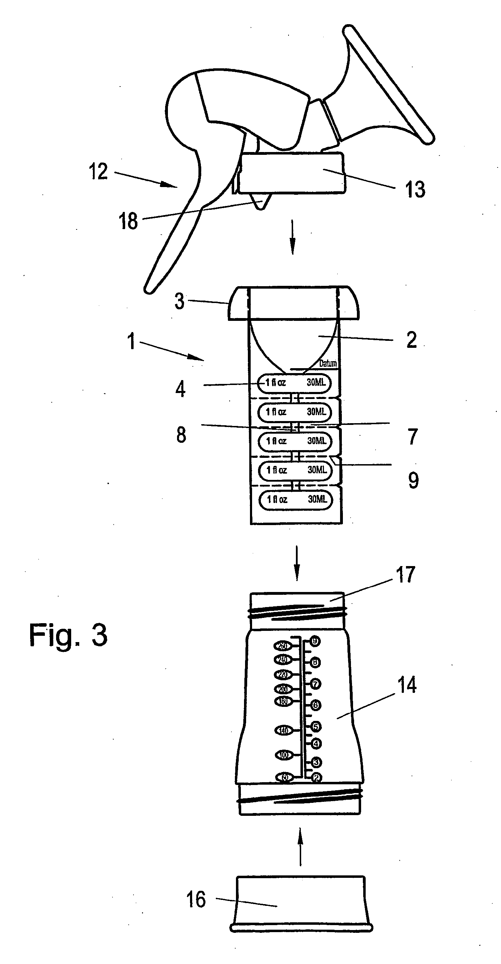

Zur Befüllung des Muttermilch-Gefrierbeutels 1 ist im oberen Abschnitt des Muttermilch-Gefrierbeutels 1 eine mittige Einfüllöffnung 2, die eine Trichterform aufweist, und ein sich über den oberen Rand des Muttermilch-Gefrierbeutels 1 erstreckender Kragen 3 vorgesehen. Wie insbesondere in Fig. 2 und Fig. 3 ersichtlich, kann der Kragen 3 umgekrempelt werden, so dass der Muttermilch-Gefrierbeutel 1 an einer Brustpumpe zum Abpumpen von Muttermilch auf einfache Weise befestigt werden kann.

Die Einfüllöffnung 2 mündet mit ihrem unteren Ende in eine erste Aufnahmeeinheit 4. Die Aufnahmeeinheit 4 mit einer Grundfläche 5 und einer Deckfläche 6 ist im Wesentlichen zylindrisch ausgebildet, wobei ihre Längsachse parallel zur Längsachse des im Wesentlichen zylindrischen Gefrierbeutels 1 angeordnet ist. Insgesamt weist der Muttermilch-Gefrierbeutel 1 fünf Aufnahmeeinheiten 4 auf, die übereinander angeordnet sind. Die voneinander beabstandet angeordneten Aufnahmeeinheiten 4 sind in Bezug auf den Muttermilch-Gefrierbeutel 1 mittig angeordnet und über Verbindungsabschnitte 7 miteinander verbunden. In den Verbindungsabschnitten 7 sind die Kunststofffolien flächig miteinander verschweißt, wobei Verbindungskanäle 8 von der Verschweißung ausgespart sind. Die Verbindungskanäle 8 erstrecken sich ebenfalls mittig von der Grundfläche 5 einer Aufnahmeeinheit 4 bis zur Deckfläche 6 der darunter angeordneten Aufnahmeeinheit 4, so dass sie die einzelnen Aufnahmeeinheiten 4 in einer vertikalen Linie kommunizierend miteinander verbinden.

Die Verbindungsabschnitte 7 sind auch im tiefgefrorenen Zustand der Muttermilch auf einfache Weise entlang den hierfür vorgesehenen Perforationen 9 durchtrennbar, so dass einzelne Aufnahmeeinheiten 4 von den übrigen Aufnahmeeinheiten 4 getrennt werden können und gesondert zum Verzehr aufgetaut werden können. Dabei ist es natürlich auch denkbar, dass die Verbindungsabschnitte 7 mittels einer Schere oder einem Stanley-Messer durchtrennt werden können, so dass Perforationen 9 nicht unbedingt notwendig sind. Zur weiteren Unterstützung der Trennung der Aufnahmeeinheiten 4 voneinander sind am Seitenrand bzw. Randabschnitt des Muttermilch-Gefrierbeutels 1, am Ende der Perforationen 7, Trennkerben 10 vorgesehen.

Die Aufnahmeeinheiten 4 zeigen an ihrer Außenfläche Markierungen, die die Volumina der Aufnahmeeinheiten 4 angeben. Bei dem gezeigten Ausführungsbeispiel weisen hierbei die Aufnahmeeinheiten 4 alle das gleiche Aufnahmevolumen auf, selbstverständlich könnten jedoch auch Aufnahmeeinheiten 4 mit unterschiedlichen Aufnahmevolumina vorgesehen sein. Weiters ist nahe dem unterem Ende der Einfüllöffnung 2 eine Aufschriftfläche 11 für die Kennzeichnung des Muttermilch-Gefrierbeutels 1 mit dem jeweiligen Abfülldatum der Milch vorgesehen.

Zum Anschluss des Muttermilch-Gefrierbeutels 1 an eine Brustpumpe 12 wird zunächst der sich vom oberen Rand der Einfüllöffnung 2 erstreckende Kragen 3 nach unten umgeklappt bzw. umgekrempelt. Wie in Fig. 3 gezeigt, weist die Brustpumpe 12 eine Überwurfkappe 13 mit einem Innengewinde auf, die auf einen Mantel 14 aufschraubbar ist. Hierbei kann ein Mantel 14 verwendet werden, wie dieser auch für eine Babyflasche einsetzbar ist. Zum Verfüttern der abgepumpten Muttermilch kann somit - nach Aufschrauben einer Bodenkappe und Befestigung eines Saugers - der gleiche Mantel 14 verwendet werden, wodurch sich die Gesamtzahl der Teile der Vorrichtungen zum Abpumpen und Füttern verringert. Am Fuß des Mantels 14 ist ein Ring 16 aus rutschfestem Material für die Standfestigkeit der Brustpumpe 12 aufschraubbar.

Zum Anschluss des Muttermilch-Gefrierbeutels 1 an die Brustpumpe 12 wird zunächst der Kragen 3 der Einfüllöffnung 2 des Muttermilch-Gefrierbeutels 1 nach außen umgeklappt (siehe Fig. 2) und anschließend der Muttermilch-Gefrierbeutel 1 in den Mantel 14 eingebracht, so dass er den oberen Rand 17 des Mantels 14 umschließt. Darauf wird die Überwurfkappe 13 der Milchpumpe 12 auf den Mantel 14 aufgeschraubt, so dass der Muttermilch-Gefrierbeutel 1 über seinen Kragen 3 am Rand 17 des Mantels 14 festgehalten wird und sich nicht ungewollt lösen kann.

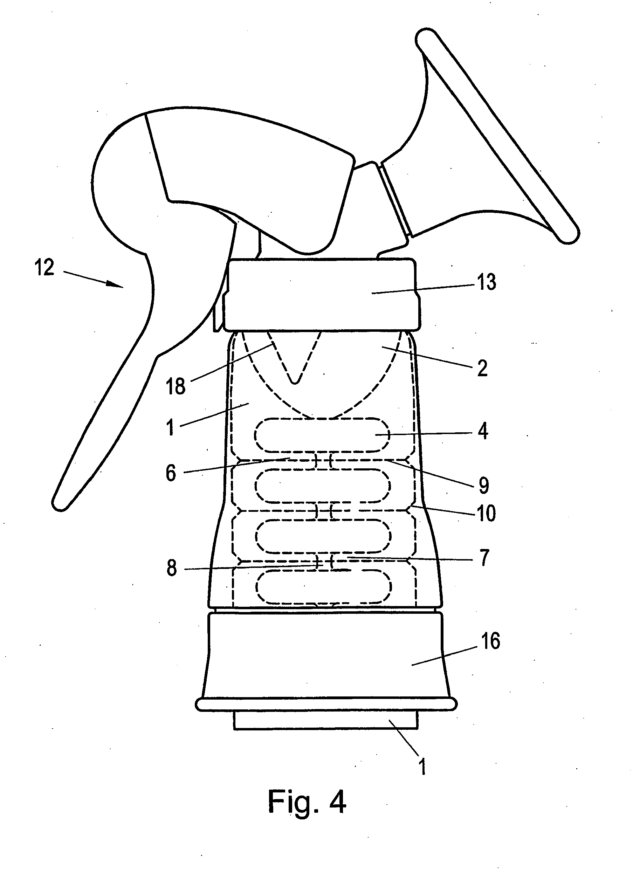

Wie in Fig. 4 ersichtlich, ragt im zusammengesetzten Zustand ein konisch geformter Auslass 18 der Brustpumpe 12 in die trichterförmige Einfüllöffnung 2 des Muttermilch-Gefrierbeutels 1 hinein. Beim Abpumpen der Milch mit Hilfe der Brustpumpe 12 gelangt die abgepumpte Milch somit direkt vom Auslass 18 der Brustpumpe 12 in die Einfüllöffnung 2, und über die trichterförmige Einfüllöffnung 2 in die oberste Aufnahmeeinheit 4.

Über die vertikal angeordneten Verbindungskanäle 8 läuft die abgepumpte Milch direkt von der ersten Aufnahmeeinheit 4 in die darunter liegenden Aufnahmeeinheiten 4.

In Fig. 4 ist weiters ersichtlich, dass der untere Abschnitt des Muttermilch-Gefrierbeutels 1 durch die mittige Öffnung im Ring 16 herausragen kann. Durch das Vorsehen eines Mantels 14 mit einem offenen Boden kann der Muttermilch-Gefrierbeutel 1 im befüllten Zustand auch eine größere Längserstreckung als der Mantel 14 aufweisen, so dass selbstverständlich auch ein Muttermilch-Gefrierbeutel mit mehr als den gezeigten fünf Aufnahmeeinheiten 4 eingesetzt werden kann. Die Anzahl der Aufnahmeeinheiten 4 des Muttermilch-Gefrierbeutels 1 kann demnach je nach Bedarf der gewünschten Aufnahmemenge des Gefrierbeutels 1 variiert werden.

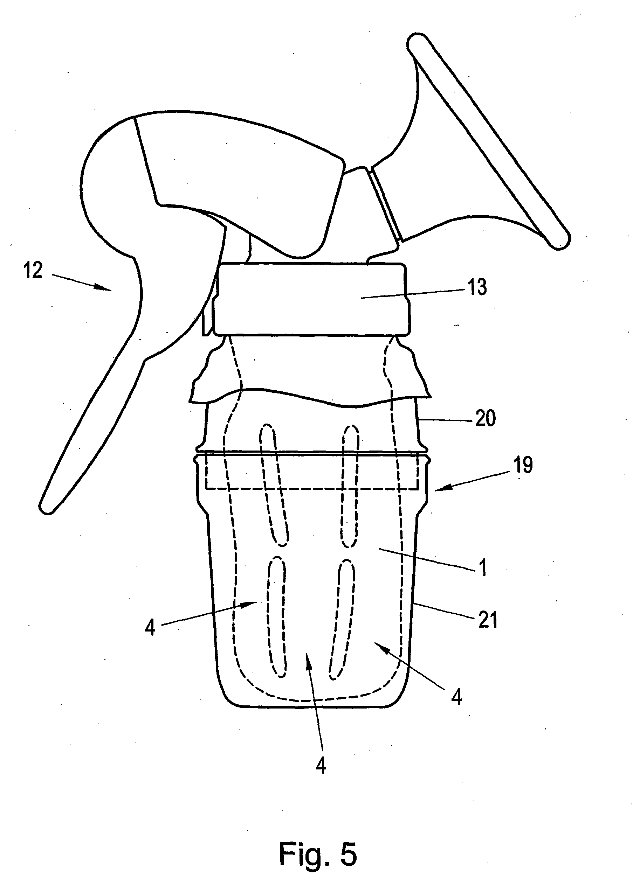

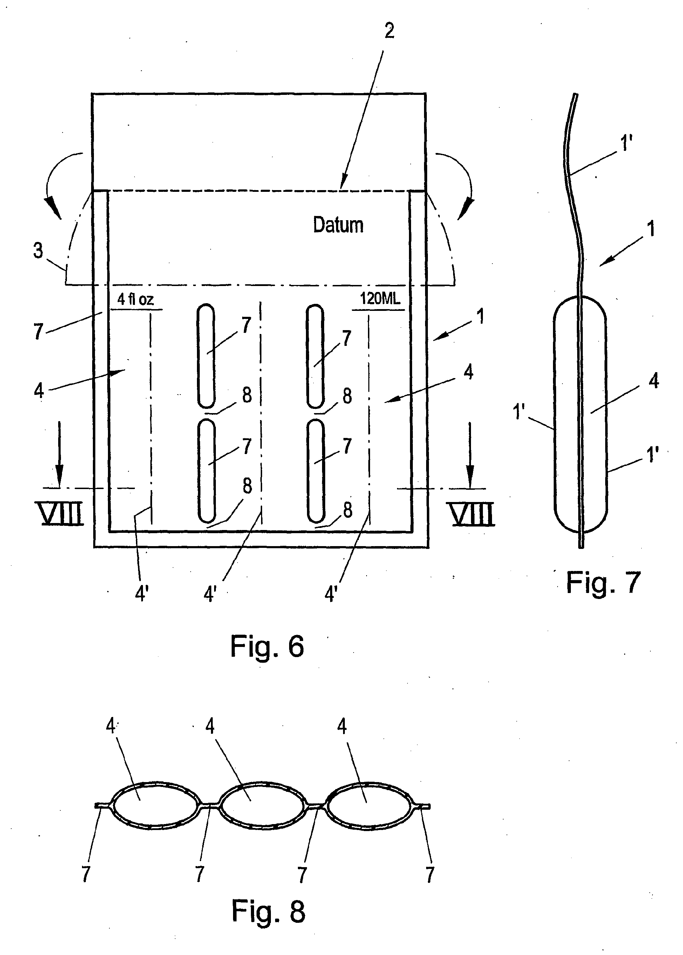

In Fig. 5 ist ein alternatives Ausführungsbeispiel des Muttermilch-Gefrierbeutels 1 gezeigt, bei welchem in der Befüllstellung seitlich nebeneinander angeordnete Aufnahmeeinheiten 4 (vgl. insbesondere auch Fig. 6 und 8) vorgesehen sind. Zudem ist an Stelle eines nach unten hin offenen Mantels 14 ein geschlossener Behälter 19 vorgesehen, der sich aus einem Oberteil 20, an welchen die Überwurfkappe 13 der Milchpumpe 12 aufgeschraubt ist, und einem Unterteil 21 zusammensetzt, wobei Ober- und Unterteil 20, 21 über eine Schraubverbindung miteinander verbunden sind.

Wie in den Fig. 6 bis 8 ersichtlich, weist der Muttermilch-Gefrierbeutel 1 mehrere Aufnahmeeinheiten 4 auf, deren Längsachsen 4' in der Befüllstellung im Wesentlichen lotrecht angeordnet sind. Der Muttermilch-Gefrierbeutel 1 besteht hierbei im Wesentlichen aus zwei Kunststofffolien 1', die in den Verbindungsabschnitten 7 flächig miteinander verschweißt sind. Um einen Übertritt von Muttermilch im flüssigen Zustand zwischen den Aufnahmeeinheiten 4 zu ermöglichen, sind die Verbindungsabschnitte 7 zwischen den Aufnahmeeinheiten 4 durch Verbindungskanäle 8 unterbrochen. Wie weiters in Fig. 6 ersichtlich, weist der Muttermilch-Gefrierbeutel 1 eine Einfüllöffnung 2 auf, welche mit allen seitlich nebeneinander angeordneten Aufnahmeeinheiten 4 zugleich in Verbindung steht.

Zudem weist der in den Fig. 5 bis 8 gezeigte alternative Muttermilch-Gefrierbeutel 1 ebenfalls so wie das Ausführungsbeispiel gemäß den Fig. 1 bis 4 einen sich über den oberen Rand des Muttermilch-Gefrierbeutel 1 erstreckenden Kragen 3 auf, der über den Oberteil 20 des Behälters 19 gekrempelt werden kann, so dass mit Hilfe der Überwurfkappe 13 der Milchpumpe 12 der Muttermilch-Gefrierbeutel 1 zuverlässig im Behälter 19 fixiert wird.

Wenn die Aufnahmeeinheiten 4 des Muttermilch-Gefrierbeutels 1 mit abgepumpter Milch gefüllt sind, kann der Muttermilch-Gefrierbeutel 1 von der Brustpumpe 12 abgenommen und in geeigneter Weise geschlossen werden. Der Muttermilch-Gefrierbeutel 1 kann mit einem Gummiring geschlossen werden, oder der Benutzer macht einen Knoten im Bereich der Einfüllöffnung 2, wonach der Gefrierbeutel 1 dicht geschlossen tiefgekühlt aufbewahrt werden kann. Bei Verwendung einer Teilmenge der abgepumpten Milch werden je nach Bedarf ein oder auch mehrere Aufnahmeeinheiten 4 des Muttermilch-Gefrierbeutels 1 im Bereich ihrer Verbindungsabschnitte 7 eventuell mit Hilfe der Perforationen 9 und Trennkerben 10 (in Fig. 4 an beiden Randabschnitten des Gefrierbeutels 1) vom Muttermilch-Gefrierbeutel 1 im gefrorenen Zustand abgetrennt. Die gefrorenen Milchportionen in den Aufnahmeeinheiten 4 können jedoch auch aus der Kunststofffolie des Muttermilch-Gefrierbeutels 1 - ohne vorherige Trennung einer Aufnahmeeinheit 4 - herausgedrückt werden und zur Verwendung der Milch in eine Babyflasche eingebracht werden, um sie für die Fütterung aufzutauen bzw. zu wärmen. Hierbei verbleibt die in dem Muttermilch-Gefrierbeutel 1 weiterhin tiefgefrorene Muttermilch im Wesentlichen (abgesehen von dem offenen Verbindungskanal) zur Umgebung durch den Gefrierbeutel 1 geschützt, so dass eine hygienische Aufbewahrung gewährleistet ist.

## Patentansprüche

1. Muttermilch-Gefrierbeutel (1) zur Aufnahme und Aufbewahrung von Muttermilch mit einer Einfüllöffnung (2), wobei die Einfüllöffnung (2) einen sich über den oberen Rand des Muttermilch-Gefrierbeutels (1) erstreckenden nach außen umklappbaren Kragen (3) zur Befestigung des Muttermilch-Gefrierbeutels (1) mit einer Brustpumpe (12) aufweist, **dadurch gekennzeichnet, dass** wenigstens zwei Aufnahmeeinheiten (4), deren Aufnahmevolumen jeweils etwa 10 ml bis 50 ml beträgt, vorgesehen sind, wobei die Aufnahmeeinheiten (4) mit jeweils zumindest einer benachbarten Aufnahmeeinheit (4) über zumindest einen Verbindungskanal (8) kommunizierend verbunden sind, so dass die in den Aufnahmeeinheiten (4) aufgenommene Muttermilch im eingefrorenen Zustand in Portionen entsprechend der Aufnahmevolumina der Aufnahmeeinheiten (4) aufteilbar ist und im aufgetauten Zustand portionsweise verfütterbar ist.

2. Muttermilch-Gefrierbeutel nach Anspruch 1, **dadurch gekennzeichnet, dass** die Aufnahmeeinheiten (4) entlang einer den Verbindungskanal (8) durchtrennenden Ebene voneinander trennbar sind.

3. Muttermilch-Gefrierbeutel nach Anspruch 2, **dadurch gekennzeichnet, dass** der Muttermilch-Gefrierbeutel (1) aus zwei Kunststoffflächen besteht, die unter Aussparung der Einfüllöffnung (2) der Aufnahmeeinheiten (4) und der Verbindungskanäle (8) flächig miteinander verschweißt sind.

4. Muttermilch-Gefrierbeutel nach Anspruch 3, **dadurch gekennzeichnet, dass** die Aufnahmeeinheiten (4) über unter Aussparung der Verbindungskanäle (8) flächig verschweißte Verbindungsabschnitte (7) miteinander verbunden sind.

5. Muttermilch-Gefrierbeutel nach Anspruch 4, **dadurch gekennzeichnet, dass** die Verbindungsabschnitte (7) Perforationen (9) aufweisen.

6. Muttermilch-Gefrierbeutel nach einem der Ansprüche 1 bis 5, **dadurch gekennzeichnet, dass** die Verbindungsabschnitte (7) in zumindest einem Randabschnitt jeweils eine Trennkerbe (10) aufweisen.

7. Muttermilch-Gefrierbeutel nach einem der Ansprüche 1 bis 6, **dadurch gekennzeichnet, dass** die Aufnahmeeinheiten (4) in der Befüllstellung des Muttermilch-Gefrierbeutels (1) übereinander angeordnet sind.

8. Muttermilch-Gefrierbeutel nach Anspruch 7, **dadurch gekennzeichnet, dass** die Aufnahmeeinheiten (4) im Wesentlichen zylindrisch ausgebildet sind.

9. Muttermilch-Gefrierbeutel nach Anspruch 8, **dadurch gekennzeichnet, dass** die Grund- und Deckflächen (5, 6) der im Wesentlichen zylindrischen Aufnahmeeinheiten (4) parallel zueinander angeordnet sind.

10. Muttermilch-Gefrierbeutel nach einem der Ansprüche 7 bis 9, **dadurch gekennzeichnet, dass** die Verbindungskanäle (8) zwischen den Aufnahmeeinheiten (4) in der Längsachse des im Wesentlichen zylindrischen Muttermilch-Gefrierbeutels (1) angeordnet sind.

11. Muttermilch-Gefrierbeutel nach einem der Ansprüche 7 bis 10, **dadurch gekennzeichnet, dass** die Einfüllöffnung (2) vom oberen Rand des Muttermilch-Gefrierbeutels (1) in die vom oberen Rand des Muttermilch-Gefrierbeutels (1) gesehen erste bzw. oberste Aufnahmeeinheit (4) mündet.

12. Muttermilch-Gefrierbeutel nach einem der Ansprüche 7 bis 11, **dadurch gekennzeichnet, dass** die Einfüllöffnung (2) trichterförmig ausgebildet ist.

13. Muttermilch-Gefrierbeutel nach einem der Ansprüche 1 bis 6, **dadurch gekennzeichnet, dass** seitlich nebeneinander angeordnete Aufnahmeeinheiten (4) vorgesehen sind, deren Längsachsen (4') in der Befüllstellung im Wesentlichen lotrecht angeordnet sind.

14. Muttermilch-Gefrierbeutel nach Anspruch 13, **dadurch gekennzeichnet, dass** die Verbindungskanäle (8) zwischen den Aufnahmeeinheiten (4) senkrecht zur Längsachse der Aufnahmeeinheiten angeordnet sind.

15. Muttermilch-Gefrierbeutel nach Anspruch 13 oder 14, **dadurch gekennzeichnet, dass** die Einfüllöffnung (2) mit allen seitlich nebeneinander angeordneten Aufnahmeeinheiten (4) in Verbindung steht.

## Claims

1. A breast milk freezer bag (1) for receiving and storing breast milk, including a filling opening (2), said filling opening (2) having a collar (3) extending over the upper edge of the breast milk freezer bag (1), and being foldable outwardly, for securing the breast milk freezer bag (1) to a breast pump (12), **characterized in that** at least two reception units (4) are provided having a reception volume of from about 10 ml to 50 ml each, wherein the reception units (4) are each connected with at least one adjacent reception unit (4) in a communicating manner via at least one connection channel (8) such that the breast milk received in the reception units (4) is dividable into portions in the frozen state as a function of the reception volumes of the reception units (4) and feedable in portions in the unfrozen state.

2. A breast milk freezer bag according to claim 1, **characterized in that** the reception units (4) are separable from one another along a plane dividing the connection channel (8).

3. A breast milk freezer bag according to claim 2, **characterized in that** the breast milk freezer bag (1) is made of two plastic surfaces which are planarly welded together while leaving free the filling opening (2), the reception units (4) and the connection channels (8).

4. A breast milk freezer bag according to claim 3, **characterized in that** the reception units (4) are interconnected by connection portions (7) which are planarly welded while leaving free the connection channels (8).

5. A breast milk freezer bag according to claim 4, **characterized in that** the connection portions (7) have perforations (9).

6. A breast milk freezer bag according to any one of claims 1 to 5, **characterized in that** the connection portions (7) each have a separation notch (10) in at least one edge portion.

7. A breast milk freezer bag according to any one of claims 1 to 6, **characterized in that** the reception units (4) are arranged one above the other in the filling position of the breast milk freezer bag (1).

8. A breast milk freezer bag according to claim 7, **characterized in that** the reception units (4) are designed in a substantially cylindrical manner.

9. A breast milk freezer bag according to claim 8, **characterized in that** the base and top surfaces (5, 6) of the substantially cylindrical reception units (4) are arranged in parallel to each other.

10. A breast milk freezer bag according to any one of claims 7 to 9, **characterized in that** the connection channels (8) between the reception units (4) are arranged in the longitudinal axis of the substantially cylindrical breast milk freezer bag (1).

11. A breast milk freezer bag according to any one of claims 7 to 10, **characterized in that** the filling opening (2) runs from the upper edge of the breast milk freezer bag (1) into the first or uppermost reception unit (4), viewed from the upper edge of the breast milk freezer bag (1).

12. A breast milk freezer bag according to any one of claims 7 to 11, **characterized in that** the filling opening (2) is designed in a funnel-shaped manner.

13. A breast milk freezer bag according to any one of claims 1 to 6, **characterized in that** laterally adjacently arranged reception units (4) are provided, whose longitudinal axes (4') are substantially vertical in the filling position.

14. A breast milk freezer bag according to claim 13, **characterized in that** the connection channels (8) between the reception units (4) are arranged in a manner perpendicular to the longitudinal axes of the reception units.

15. A breast milk freezer bag according to claim 13 or 14, **characterized in that** the filling opening (2) communicates with all of the laterally adjacently arranged reception units (4).

## Revendications

1. Sachet de congélation pour lait maternel (1) pour le recueillement et la conservation de lait maternel avec une ouverture de remplissage (2), l'ouverture de remplissage (2) présentant une collerette (3) s'étendant sur le bord supérieur du sachet de congélation pour lait maternel (1) et rabattable vers l'extérieur pour l'attache du sachet de congélation pour lait maternel (1) avec une pompe pour le sein (12), **caractérisé en ce qu'**au moins deux unités de recueillement (4), dont le volume de recueillement est respectivement d'environ 10 ml à 50 ml, sont prévues, les unités de recueillement (4) étant reliées chacune à au moins une unité de recueillement (4) voisine en communiquant au moyen d'au moins un canal de liaison (8), de sorte que le lait maternel recueilli dans les unités de recueillement (4) peut être subdivisé à l'état congelé en portions en fonction des volumes de recueillement des unités de recueillement (4) et peut être donné à boire par portions à l'état dégelé.

2. Sachet de congélation pour lait maternel selon la revendication 1, **caractérisé en ce que** les unités de recueillement (4) peuvent être séparées les unes des autres le long d'un plan séparant le canal de liaison (8).

3. Sachet de congélation pour lait maternel selon la revendication 2, **caractérisé en ce que** le sachet de congélation pour lait maternel (1) est constitué de deux surfaces plastiques qui sont soudées en surface l'une à l'autre en évidant l'ouverture de remplissage (2) des unités de recueillement (4) et des canaux de liaison (8).

4. Sachet de congélation pour lait maternel selon la revendication 3, **caractérisé en ce que** les unités de recueillement (4) sont reliées les unes aux autres au moyen de parties de liaison (7) soudées en surface en évidant les canaux de liaison (8).

5. Sachet de congélation pour lait maternel selon la revendication 4, **caractérisé en ce que** les parties de liaison (7) présentent des perforations (9).

6. Sachet de congélation pour lait maternel selon l'une quelconque des revendications 1 à 5, **caractérisé en ce que** les parties de liaison (7) présentent chacune une entaille de séparation (10) dans au moins une partie de bordure.

7. Sachet de congélation pour lait maternel selon l'une quelconque des revendications 1 à 6, **caractérisé en ce que** les unités de recueillement (4) sont disposées les unes au-dessus des autres dans la position de remplissage du sachet de congélation pour lait maternel (1).

8. Sachet de congélation pour lait maternel selon la revendication 7, **caractérisé en ce que** les unités de recueillement (4) sont conçues de façon sensiblement cylindrique.

9. Sachet de congélation pour lait maternel selon la revendication 8, **caractérisé en ce que** les surfaces de fond et de recouvrement (5, 6) des unités de recueillement (4) sensiblement cylindriques sont disposées parallèlement les unes aux autres.

10. Sachet de congélation pour lait maternel selon l'une quelconque des revendications 7 à 9, **caractérisé en ce que** les canaux de liaison (8) sont disposés entre les unités de recueillement (4) dans l'axe longitudinal du sachet de congélation pour lait maternel (1) sensiblement cylindrique.

11. Sachet de congélation pour lait maternel selon l'une quelconque des revendications 7 à 10, **caractérisé en ce que** l'ouverture de remplissage (2) débouche par le bord supérieur du sachet de congélation pour lait maternel (1) dans la première unité de recueillement (4), vu depuis le bord supérieur du sachet de congélation pour lait maternel (1), ou dans l'unité de recueillement (4) supérieure.

12. Sachet de congélation pour lait maternel selon l'une quelconque des revendications 7 à 11, **caractérisé en ce que** l'ouverture de remplissage (2) est conçue en forme d'entonnoir.

13. Sachet de congélation pour lait maternel selon l'une quelconque des revendications 1 à 6, **caractérisé en ce qu'**il est prévu des unités de recueillement (4) disposées sur le côté les unes à côté des autres, dont les axes longitudinaux (4') sont disposés sensiblement verticalement dans la position de remplissage.

14. Sachet de congélation pour lait maternel selon la revendication 13, **caractérisé en ce que** les canaux de liaison (8) sont disposés entre les unités de recueillement (4) perpendiculairement à l'axe longitudinal des unités de recueillement.

15. Sachet de congélation pour lait maternel selon la revendication 13 ou 14, **caractérisé en ce que** l'ouverture de remplissage (2) est en liaison avec toutes les unités de recueillement (4) disposées latéralement les unes à côté des autres.
